# EUROPEAN PATENT APPLICATION

(11) **EP 0 540 194 A2**
(43) Date of publication of application: **05.05.1993**
(21) Application number: 92309119.3
(22) Date of filing: 07.10.1992
(51) Int. Cl.: A61F 13/06

(54) **Callous pad**

(30) Priority: 18.10.1991 GB 9122266
(71) Applicant: SCHOLL PLC, Windsor, Berkshire SL6 1BG (GB)
(72) Inventor: Press, Robert Christopher Stuart, Alton, Hampshire GU34 1JQ (GB)
(74) Representative: Purvis, William Michael Cameron

(57) **Abstract**

A callous pad formed of felt or foam has a removable middle portion (5) which is mostly separated from a surrounding outer portion (6) by a cutline (7) but is retained by spigots (8) which can be broken or cut as desired to allow the middle portion (5) to be removed.

## Description

The invention relates to a callous pad.

Callous pads, sometimes referred to as callous plasters, have been previously proposed and traditionally comprised a pad of pressure absorbing material, such as felt or a foam, with an adhesive face which could be adhered to a foot of a person suffering from callouses to pad the callous and relieve aggravation caused by pressure or friction of a shoe worn on the foot. Such a callous pad might be a solid pad or alternatively formed as an annulus which could be placed over a tender area so that the tender area projected into the middle of the annulus and was protected against pressure.

According to the invention a callous pad comprising a pad of a pressure absorbing material with adhesive provided on one face thereof characterised in that the perimeter of a middle portion is cut through so as mostly to separate the middle portion from a surrounding portion but leave relatively weak retaining portions.

Thus, a callous pad according to the invention can, at the option of the user, be applied either as a continuous solid pad or as a generally annular pad merely by either retaining the middle portion or separating it out from the surrounding portion by snipping or pressing and tearing.

Preferably the middle portion is of circular form but could be of other form if preferred. The exterior shape of the pad may also if desired be circular, preferably concentric with the middle portion, but may be of other form, for example generally rectangular, preferably square, with rounded off corners and with one of the sides concave.

Foam and felt are both suitable materials for forming the callous pad of the invention and the outer face, that is say the face opposite to the face with the adhesive thereon could be of any colour but is preferably generally skin coloured so that the pad is inconspicuous in use.

If the callous pad is formed of felt it is preferably a flesh coloured needle felt which is laminated to a white cotton textile, the cotton textile being coated with the adhesive. Preferably the adhesive is an acrylic adhesive. The cotton textile could be omitted but is preferably included to assist processing. The textile could alternatively be synthetic for example polyester.

If the callous pad is formed of foam it can be formed with a single layer or with multiple layers, and with open or closed cells. It may for example be polyethylene, polyurethane or natural or synthetic rubber, but is preferably a styrene-butadiene-rubber:natural rubber latex rubber foam comprising two layers of differing densities spread onto a fabric. The fabric or one surface of the foam is coated with the adhesive and the top surface of the foam is spread or laminated with a flexible polyurethane coating for example polyurethane, acrylic, to increase ultra violet stability and to provide a slippery surface. The callous pads may be mounted on a release paper coated with clay, silicone or polyethylene as well known in the art.

Each middle portion is preferably completely cut out from the surrounding portion except for up to six, preferably three to four and ideally three small bridging portions mutually spaced ideally at 120° for three bridging portions. In a felt embodiment the bridging portions are preferably cut through with scissors if the middle portion is to be removed but in a foam embodiment the middle portion can advantageously be torn from the surrounding portion without requiring the use of scissors.

The invention is diagrammatically illustrated by way of example in the accompanying drawings in which:
Figures 1a, 1b and 1c are successive perspective views showing one embodiment of a callous pad according to the invention with a middle portion engaged therein, partially removed therefrom and fully removed therefrom respectively;
Figure 1d is a greatly enlarged fragmentary view of a portion of Figure 1a;
Figure 2 is a plan view of a card bearing a plurality of the callous pads of the embodiment of Figures 1a to 1d;
Figure 3 is a sectional view on line IV-IV of Figure 3;
Figure 4 is a view corresponding to Figure 2 but Showing a second embodiment of a callous pad according to the invention; and
Figure 5 is a sectional view on V-V of Figure 6.

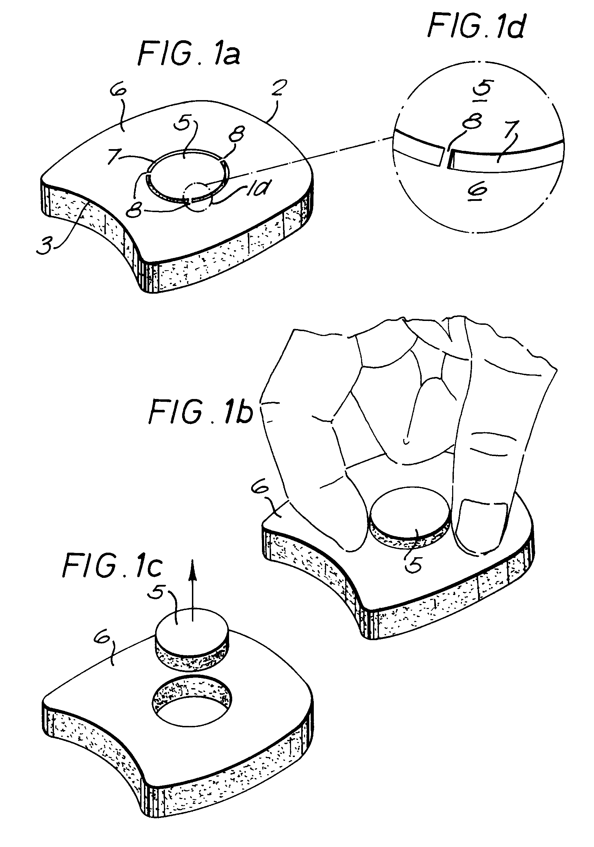

Referring to the drawings and firstly to Figures 1a to 1d, 2 and 3, a callous pad 1 is generally square in plan view with rounded corners 2 and a concave side 3.

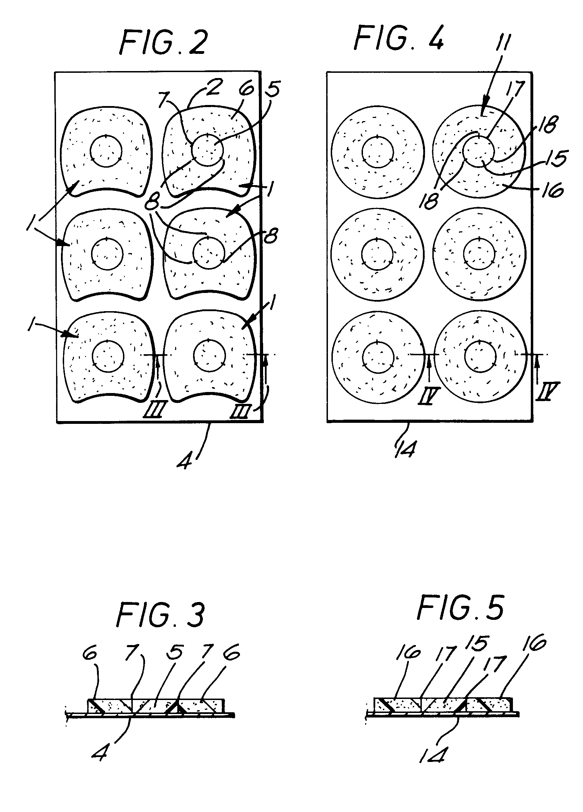

The pad 1 may be approximately 34mm wide, 33.5mm high and the concavity of the side 3 may be 3mm. As shown in Figure 3 it may be 3.5mm thick and mounted with five others on a card 4 which is 0.15mm thick. The pad 1 has a circular middle portion 5 of 11.5mm in diameter. The middle portion 5 is separated from a surrounding portion 6 by a cutline 7 which extends for the full thickness of the pad 1 but which is discontinuous in that it has three breaks therein mutually at 120° forming spigots 8 which are 0.5mm wide and which secure the middle portion 5 to the surrounding portion 6.

In the embodiment of Figures 4 and 5, a pad 11 is of circular form and is mounted with five other identical pads on a mounting card 14. It has a circular middle portion 15 the perimeter of which is formed by a cutline 17 and which separates it from an outer portion 16 the perimeter of which is concentric with the cutline 17. The cutline 17 has three breaks therein which form spigots 18 connecting the middle portion 15 to the surrounding portion 16. The pad 11 is 34mm in diameter, the middle portion 5 is 11.5mm in diameter and the whole pad is 3.5mm thick and is mounted on a 0.15mm thick card 14.

A person requiring a callous pad can thus, while only needing to purchase a single form of pad, have the option of using the pad as a continuous pad or as an annular pad indeed it is possible in use to changeover from using a solid pad to using a annular pad merely by removing the middle portion 5 or 15.

If the pads 1 and 11 are formed of felt rather of foam then they preferably have a thickness of 4mm rather than 3.5mm.

The pressure absorbing material could be up to 6mm in thickness or as thin as 2mm but is preferably between 3 and 4mm in thickness.

## Claims

1. A callous pad comprising a pad (1, 11) of a pressure absorbing material with adhesive provided on one face thereof characterised in that the perimeter of a middle portion (5, 15) is cut through so as mostly to separate the middle portion (5, 15) from a surrounding portion (6, 16) but leave relatively weak retaining portions (8, 18).

2. A callous pad according to claim 1, in which the perimeter of the middle portion (5, 15) is circular.

3. A callous pad according to claim 1 or claim 2, in which the exterior perimeter of the pad (11) is circular and concentric with the perimeter of the middle portion (15).

4. A callous pad according to claim 1 or claim 2, in which the perimeter of the pad (1) is generally rectangular with rounded off corners (2) and with one (3) of the sides concave.

5. A callous pad according to any one of claims 1 to 4, formed of a flesh coloured needle felt laminated to a textile, the textile being coated with adhesive.

6. A callous pad according to any one of claims 1 to 5, in which the adhesive is an acrylic adhesive.

7. A callous pad according to any one of claims 1 to 4, formed of foam.

8. A callous pad according to claim 7, in which the foam is a styrene-butadiene-rubber:natural rubber latex rubber foam comprising two layers of differing densities spread onto a fabric.

9. A callous pad according to claim 8, in which the fabric or one surface of the foam is coated with the adhesive and the top surface of the foam is spread or laminated with a flexible polyurethane coating.

10. A callous pad according to any one of the preceding claims, mounted on a release paper (4, 14).

11. A callous pad according to any one of the preceding claims, in which the middle portion is cut out from the surrounding portion by a cut line (7, 17) which extends for the full periphery except for three small bridging portions mutually spaced at 120° and forming the weak retaining portions (8, 18).
